Europäisches Patentamt

(19) European Patent Office

Office européen des brevets

(11) Publication number: **0 064 393**
**A2**

# (12) EUROPEAN PATENT APPLICATION

(21) Application number: **82302211.6**

(22) Date of filing: **29.04.82**

(51) Int. Cl.³: **A 61 M 1/03**

(30) Priority: **04.05.81 US 259793**

(43) Date of publication of application:
**10.11.82 Bulletin 82/45**

(84) Designated Contracting States:
**AT BE CH DE FR GB IT LI LU NL SE**

(71) Applicant: **PURDUE RESEARCH FOUNDATION**
**Graduate House East**
**West Lafayette Indiana 47907(US)**

(72) Inventor: **Ash, Stephen Richard**
**2500 N. County Road 400 East**
**Lafayette Indiana 47905(US)**

(74) Representative: **Hudson, Christopher Mark**
**Erl Wood Manor**
**Windlesham Surrey GU20, 6PH(GB)**

(54) Sorbent mixture for use in hemodialysis.

(57) This invention concerns a sorbent mixture for a sorbent suspension reciprocating dialyzer comprising a surface adsorptive agent capable of adsorption of uremic substances, a cation exchanger, urease, an aliphatic carboxylic acid resin in the H⁺ form, water and a suspending agent.

EP 0 064 393 A2

Croydon Printing Company Ltd.

X-5508                                    -1-

## SORBENT MIXTURE FOR USE IN HEMODIALYSIS

This invention concerns a sorbent mixture for a sorbent suspension reciprocating dialyzer comprising a surface adsorptive agent capable of adsorption of uremic substances, a cation exchanger, urease, an aliphatic carboxylic acid resin in the $H^+$ form, water and a suspending agent.

A sorbent suspension reciprocating dialyzer (SSRD), a parallel plate dialyzer with a single blood access, is disclosed and claimed in United States Patent 4,071,444 issued January 31, 1978. This dialyzer contains a stack of resilient semi-permeable membranes separated by gaskets and spacers which permit blood to be introduced through a central core bolt. This stack of resilient semi-permeable membranes is placed inside a sealed container known as a dialysate chamber. The dialysate chamber contains as a sorbent mixture a combination of water, activated charcoal, zirconium phosphate, zirconium oxide and urease.

Belgium Patent No. 874,429 issued August 27, 1979, discloses and claims sorbent mixtures for use in an SSRD. The sorbent mixtures disclosed comprise charcoal, urease and a cation exchanger, preferably a zeolite which is calcium loaded, and a suspending agent. In a preferred aspect of that invention, a zeolite or zeolite mixture which is about 50% calcium loaded, with the other exchange sites being taken up by sodium and potassium, is used.

It has now been discovered that a composition comprising:

a surface adsorptive agent capable of adsorption of uremic substances;

water;

a suspending agent;

a cation exchanger;

an aliphatic carboxylic acid resin in the $H^+$ form; and

urease is useful for dialysis.

It has additionally been discovered that a method for the adsorption of uremic substances in an artificial kidney comprises:

suspending with a suspending agent in water a mixture of components that includes a surface adsorptive agent capable of adsorption of uremic substances, urease, a cation exchanger and an aliphatic carboxylic acid resin in the $H^+$ form;

contacting one side of said membranes with said suspension and the other side of said membrane with blood whereby uremic substances in said blood are dialyzed therefrom and then removed by said suspended mixture of components.

This novel sorbent composition is particularly useful in a parallel plate single needle dialyzer of the type described in U.S. patent 4,071,444. The sorbent mixture of this invention would replace the dialysis material (16) in the chamber (15). (This prior dialysis material consists of water, activated charcoal, zirconium phosphate, zirconium oxide and urease). In such a machine, blood from the patient passes into a chamber in which are a stack of membranes with the dialysis material or sorbent in a second chamber on the other side of the membrane. Various toxins in the blood, particularly urea and creatinine, are dialyzed; ie, cross the membrane into the sorbent mixture which must be able to remove these toxins, or at least reduce their concentration to a level which will promote continued toxin removal across the membranes during dialysis.

In general, the above toxins are small organic molecules and are in greater concentration on the blood side of the dialysis membrane than on the sorbent side. As a consequence, those toxins which can cross the membrane do so until an equilibrium is reached. In a standard dialysis apparatus (kidney machine), attainment of equilibrium is prevented by "washing away" the toxins; ie, flowing water in large volume past the membrane and into a drain. Some kidney machines provide for column purification of the water which can then be recirculated. Obviously, in a portable, self-contained, single-needle dialyzer, there is no possibility of simply avoiding an equilibrium situation by flushing out the dialyzed toxins as they cross the membrane; purification and reuse of the dialysis water is a necessity. The same considerations apply to any portable device to be used with recently developed continuous ambulatory peritoneal dialysis.

Many organic toxins, such as creatinine, can be removed from the dialysate by adsorption on a highly surface-active material such as silica. I prefer to use activated charcoal as the non-specific, surface-active component of the sorbent mixture. The toxin present in largest amounts in patients with kidney failure is, of course, urea and it is one of the chemicals dialyzed in greatest quantity. Urea is decomposed by urease as it passes into the dialysate to give one mole of carbon dioxide (as bicarbonate or carbonate) and two moles of ammonia (or ammonium ions) per mole of urea. Both of these derived materials, carbonate and ammonium ions, are now in greater concentration on the dialysate side of the membrane than on the blood side and will pass through the membrane and into the blood unless removed. Removal of $NH_4^+$ can be effected by contact with zirconium phosphate or the $Na^+$ and $K^+$ form of an ion-exchange resin. However, since the $NH_4^+$ must replace another ion in order to be removed from the dialysate by the ion-exchanger, the released ion will now be in excess on the dialysate side and will pass into the blood stream. Thus, if the resin is in the $Na^+$ or $K^+$ form, excess (above normal for blood) $Na^+$ or $K^+$ will be generated and these ions will pass into the blood stream. The resultant excess $Na^+$ and $K^+$ in blood will cause undesirable side effects. Thus, the removal system must also operate to remove these tertiary products before they can pass the membrane and enter the blood stream. Similarly, if an acid form of a resin is used, the $H^+$ produced by $NH_4^+$ absorption will cause acidosis on passing into the blood. Likewise, the $CO_3^=$ produced by the action of urease on urea will result in excess carbonate or bicarbonate being passed into the blood stream with consequent alkalosis. I prefer to minimize the ion production problem by using a highly-calcium loaded zeolite in which about half the available replacement sites are filled by $Ca^{+2}$ and the other half by $Na^+$ (except that a few may be

X-5508        -5-

filled with $K^+$ ions if desired). Very highly-calcium loaded artificial zeolites (about 75% replaceable sites) are available from Union Carbide Corporation, Linde Division, Tarrytown, N.Y. These are prepared by forming the zeolite in a system containing a high proportion of $Ca^{+2}$ (rather than $Na^+$ or $H^+$ ions). These very highly-calcium loaded zeolites will contain about 4.6 meq/gr. of $Ca^{+2}$ and about 1.5 meq/gr. $Na^+$. Ordinarily, to prepare a 50% $Ca^{+2}$-loaded resin, I titrate the 75% $Ca^{+2}$-loaded resin with a $Na^+$ solution (hydroxide or carbonate) until the zeolite has about a 50-50 $Ca^{+2}$-$Na^+$ loading. Alternatively, I can add a second zeolite with a sodium-potassium loading such that the final zeolite mixture has about 50% available (or replaceable) $Ca^{+2}$ and 50% $Na^+$-$K^+$. The single zeolite or a mixture of two or more zeolites should contain, per gram, 2.8-3.2 meq $Ca^{+2}$ (preferably about 3.0 meq $Ca^{+2}$), 2.3-3.0 meq $Na^+$ (preferably about 2.5 meq $Na^+$) and from 0 to 1 meq $K^+$ (preferably about 0.5 meq $K^+$). A small amount of zeolite, either the calcium loaded or the sodium-potassium loaded will have urease bound to it as will be set forth hereinafter.

The advantage of using a highly-calcium loaded zeolite lies in the fact $Ca^{+2}$ can react with carbonate (via the carbonate-bicarbonate-$CO_2$ equilibrium) to form calcium carbonate which is relatively insoluble in water (solubility product constant = $8.7 \times 10^{-9}$ at room temperature). Thus, not only is $NH_4^+$ removed by displacing $Ca^{+2}$ from the zeolite but also only a small amount of the $Ca^{+2}$ thus displaced is available for transport back across the dialysis membrane into the blood. In addition, much of the $CO_2$ produced by the urease is precipitated as $CaCO_3$ and is not available to enter the blood stream as bicarbonate ion to produce alkalosis.

The urease member of my novel dialysis combination may be present as such at a concentration of about 20 international units/ml. Preferably, however, the urease can be bound to a zeolite as follows:

500 g. of calcium-exchanged Ionsiv w (a zeolite obtained from Union Carbide) were ground in a mortar and then dried at 55°C. for about 48 hours. The ground zeolite was suspended in 2000 ml. of a 12% (v/v) solution of gamma-aminopropyltriethoxysilane in toluene. The reaction mixture was stirred overnight at about 95°C. and was then filtered through Whatman No. 1 paper. The filtered zeolite was washed three times by suspending the filter cake in 500 ml. of toluene, slurrying and then refiltering. The filtered zeolite was washed twice more in the same manner with 500 ml. of acetone, except that the filter cake was collected on Whatman No. 4 paper. Next, the filter cake was washed three times in the same way with 900 ml. of a 0.05M sodium bicarbonate solution containing 0.001M calcium chloride using Whatman No. 4 paper. The washed zeolite was then suspended in 1 liter of the same bicarbonate-calcium chloride solution and held overnight at 4°C. Next an additional 6500 ml. of the above bicarbonate-calcium chloride solution was added to the slurry. Sufficient glutaraldehyde was added to give a final concentration of 2.5% (v/v). The reaction mixture was stirred at ambient temperature for about three hours and the derivatized zeolite was separated
by filtration on Whatman No. 1 paper.  The filter cake
was washed five times by suspension in 900 ml. of the
same bicarbonate-calcium chloride solution and then
refiltered using Whatman No. 4 paper.  A Tollens test
for aldehyde showed faintly positive.  Next, crude
urease was dissolved in 2000 ml. of the same bicarbonate-calcium chloride solution in sufficient quantity
to give a 10 mg./ml. concentration.  The urease solution was filtered through a Cuno Zeta$^+$ filter.  The
washed zeolite obtained as above was suspended in this
solution and the resulting mixture stirred for two
hours at ambient temperature.  The mixture was then
held at 4°C. overnight.  The zeolite containing urease
bound thereto was recovered by filtration on Whatman
No. 1 paper and was washed five times with 900 ml. of
the same bicarbonate-calcium chloride solution by the
process previously outlined and the zeolite was recovered by filtration on Whatman No. 4 paper.  The
Tollens aldehyde test was negative after the second
wash.  After the final wash, the wet urease-zeolite was
suspended in sufficient of the same bicarbonate-
calcium chloride solution to give a final total volume
of 1400 ml.  The suspension had the following characteristics:  Total solids, 0.3313 g./ml., urease activity,
110 mu/ml.; total units of urease activity bound to
zeolite equals 67% of total starting units.

        The zeolite having urease bound thereto was
recovered by filtration and dried.  Total yield = 463.8 g.

        Urease is bound to zeolites F80 and W85,
Phillipsite or Clinoptilite in the calcium-loaded form by the same procedure. In such case, the urease-bound calcium-loaded zeolite is substituted for part of the total calcium-loaded zeolite needed.

Before binding to a zeolite, urease must be solubilized, and toxic components minimized. One way of purifying urease is as follows: Jack bean meal is extracted with water (400 g. with 20 liters of glass-distilled water). The mixture is stirred at room temperature for thirty minutes, and then centrifuged at 10,000 x g for thirty minutes at 4°C. The supernatant is freeze-dried in stainless steel pans in the vacuum oven at 30°C. The yield of crude urease from this amount of meal is approximately 100 g. with a specific activity of approximately 30 $\mu$/mg of protein. The average yield of enzyme is about 3300 units/gram of meal.

Next, 30 g. of crude urease is dissolved in 3.0 l. of sterile 0.1 M, pH 7 phosphate buffer containing 0.1 M NaCl and 0.001 M Na$_2$EDTA. The cloudy solution is clarified by pressure filtration through a Cuno Zeta-plus filter cartridge. In addition to clarifying the solution, this filter media also removes some of the endotoxins present in the crude extract. This solution is then stirred for 90 minutes at room temperature with 50 g. dry weight equivalent of activated thiol-Sepharose 4B which had been previously swollen and equilibrated with the same buffer. Activated thiol-Sepharose 4B is a mixed disulfide formed between 2,2'-dipyridyl disulfide and glutathione coupled to CNBr-activated Sepharose 4B. After binding the urease, the mixture is filtered under suction on a medium porosity sintered glass funnel. The Sepharose
is then washed in place on the funnel by resuspending
three times in 250 ml. of the pH 7 buffer. It is next
equilibrated with sterile pH 8, 0.1 M phosphate buffer
containing 0.1 M NaCl and 0.001 M $Na_2EDTA$. A total of
approximately 1.0 l. of the buffer is used in multiple
washes to effect the equilibration. The urease is then
eluted by stirring with 360 ml. of the pH 8 buffer made
0.025 molar in L-cysteine at room temperature for 30
minutes. The resulting mixture is filtered on a
sintered glass funnel, and the elution repeated a total
of five times. The first three eluates contain 96-98%
of the total elutable urease, and these are added to a
final concentration of 0.1 mg/ml to help stabilize the
urease. This solution is then dialyzed in Visking
tubing for twenty hours, with stirring, at 4°C. against
25 volumes of 0.1 M NaCl to remove the cysteine,
cystine, and most of the phosphate. The final dialyzed
eluate is then used as the source of urease in the
slurry for a dialysate used in a self-contained artificial kidney or for binding to a zeolite.

The $Ca^{++}$ and base generated are too high for
certain uremic patients with alkalosis or hypercalcemia.
In order to alleviate this possible alkalosis as hypercalcemia a second type of ion-exchange resin in the $H^+$
form in which aliphatic carboxylic acid groups are
the ion exchangers is present. A useful type of aliphatic carboxylic acid resin is prepared by polymerizing methacrylic acid and cross-linking the polymer thus
produced with divinylbenzene. Such resins are obtainable commercially under the trade names Bicrex-70, (Biorad Labs), Zeocarb 226 (Permutit Co), and Amberlite IRP-64 and IRP-64M, (Rohm and Haas) (these latter resins are essentially anhydrous pulverized forms of IRC-50 and have been screened or sieved to give a more uniform particle size than that present in the commercial resin). These resins are in the $H^+$ form with IRP-64 being a 100 mesh material with 30% maximum retention on a 200 mesh screen, the remainder of the material between roughly 200-400 mesh. IRP-64M is a 325 mesh product with 90% passing that size screen. Such resins are referred to as "pharmaceutical grade" resins.

A suspending agent is preferably present in my sorbent mixture. Useful suspending agents include dextran, hydroxyethyl starch and methyl cellulose. The suspending agent should be non-dialyzable, and I prefer to use methyl cellulose at a concentration in the sorbent mixture in the range 0.1-1%, usually about 0.5%. It should be recognized that the entire sorbent mixture will not be in true permanent suspension, and that, in time, the activated charcoal and zeolites will settle out. However, the degree of suspension is sufficient to maintain a mobile sorbent mixture which by the movement of the membranes is kept in sufficient motion to continually present fresh sorbent to the dialysis membrane. By this expedient, layering or packing of the sorbent constituents against the dialysis membrane is inhibited. (Such packing slows the rate of transfer of toxins from blood a saturation sorbents proximate to the dialysis membraneoccurs and diffusion distance to unsaturated sorbent increases.

The organic buffers which may permissably be for titrating generated $HCO_3-C$ for use when alkalosis preexists in the patient must be metabolizable since these organic acids or ions are capable of passing through the dialysis membrane.  Such metabolizable acids or acetic, citric and tartaric can be used but, in general, I prefer to use acids with greater buffering power permit weight such as lactic and diethylmalonic acids.

Another feature of my sorbent mixture which must be controlled is particle size.  It is apparent that smaller particle sizes will yield a better suspension and that overall efficiency will be increased by increasing surface area of sorbents and by minimizing diffusion distances required to reach or penetrate sorbent particles, such as the time required for an $NH_4^+$ ion to find a site on the zeolite carrying $Ca^{+2}$ or $Na^+$, or for a urea molecule to encounter a coupled urease molecule.  However, I have found that further pulverizing the commercially available zeolites set forth above can lead to undesired effects, such as aggregation.  The commercially available zeolites are composed of particles 1-10 microns in diameter and varying in shape from roughly spherical to cuboidal. To measure the propensity of a particularly sized resin or zeolite to aggregate, I employ an equation involving rate of flow $T=V^m$ wherein T is time and V volume as measured in a gravity flow meter (see Barile et al, AICE Abstracts-Chicago Meeting November 16-20, 1980). The exponent "m" is determined empirically, is always greater than one, but should be as close to one as possible. Determinations of "m" have enabled myself and coworkers to determine which sorbent slurries will not aggregate in a 4-hour period, that being the estimated time for daily dialysis using the SSRD.

A dialysis using the SSRD is carried out as follows: an aqueous sorbent mixture is prepared containing 7 weight percent powdered activated charcoal, 20 weight percent calcium-sodium (approximate 50-50) loaded Ionsiv W (from Union Carbide), urease bound covalently thereto (20 units per ml. of sorbent suspension), 0.5% methyl cellulose, and 3% glucose. Sodium bicarbonate (44 millimolar) is added and the pH adjusted to 5.5-5.8 if necessary. A carboxylic acid exchange resin in $H^+$ form is added at a rate of 10-40 grams per liter of sorbent mixture. The sorbent suspension is then placed in a rigid case surrounding the dialyzer membrane package after the blood compartment is injected with Betadine (PVP-iodine). Sterile irrigation fluid is rinsed into and out of the blood compartment. Several cycles are required to remove the light brown color resulting from the PVP-iodine losing iodine by dilution and transfer across the membrane. The dialyzer blood inlet port is next attached to the A-V shunt (carotid artery, jugular vein) of a mongrel dog who has been subjected to a left nephrectomy and one of whose arteries to the right kidney had been tied off. A pressure-vacuum pump is attached to a reservoir which is attached to the dialysate case. A timer controls the pressure and vacuum on a predetermined cycle (usually 42 sec. inflow, 30 sec. outflow). Heparin is introduced into the blood pathway to prolong the activated clotting time, (1000-2000 units initially). Provision is made for sampling the inflowing and exiting blood usually through a rubber port which may be punctured by a needle. The dialysis is then started by activating the pump and continued for as long as desired.

Using the above sorbent system, there is little or no base (bicarbonate) or calcium exchange in most patients, while urea, $NH_4^+$, creatinine etc. are effectively removed as before. However, for some patients on dialysis, bicarbonate return is still too high. To diminish this return to nearly zero, I add an organic buffer such as lactic or diethylmalonic acid at about 15 grams/liter of dialysate. Both buffers are metabolizable by the body. The pH of the slurry when diethylmalonic acid was used with the zeolites was 7.2 at the start of dialysis.

As previously stated, the 50-50 calcium-sodium zeolite can be replaced by a two to one mixture of a 75-25 calcium-sodium zeolite (Ionsiv W) plus a 90%-10% sodium potassium zeolite (Ionsiv F).

The above dialyzer with my novel sorbent system is able to effect a 50% removal of urea at a 22 g./l blood level on each cycle and creatinine at a 0.2 g./l level with a negligible calcium, sodium or bicarbonate return (0-5 meq/l. inflow-outflow difference).

My novel sorbent mixture has been illustrated with regard to an SSRD machine, but it is apparent that

X-5508                    -14-

it would be equally useful in a hollow filter dialyzer
or in peritoneal dialysis with a suitable dialyzer
being used to cycle peritoneal dialysate into and out
of the abdomen.

## Claims

1. A composition for use in dialysis comprising:

a surface absorptive agent capable of adsorption of uremic substances;

water;

a suspending agent;

a cation exchanger;

an aliphatic carboxylic acid resin in the $H^+$ form;

and urease.

2. A composition as claimed in claim 1 wherein said surface absorptive agent is charcoal.

3. A composition as claimed in claims 1 or 2 wherein said suspending agent is methylcellulose, hydroxyethyl starch, or dextran.

4. A composition as claimed in any one of claims 1-3 wherein the cation exchanger is one of Ionsiv F-80, Ionsiv W-85, phillipsite or clinoptilolite.

5. A composition as claimed in any one of claims 1 to 4 wherein the cation exchanger is a calcium-loaded zeolite cation exchanger.

6. A composition as claimed in any one of claims 1 to 5 in which the calcium loading of the zeolite is about 50%.

7. A composition as claimed in any one of claims 1 to 6 wherein a mixture of a calcium-loaded zeolite and a sodium or sodium-potassium-loaded zeolite is used such that the exchangeable calcium ions per gram of zeolite is in the range of 2.8-3.2 milliequivalents.

8.   A composition as claimed in any one of claims 1 to 7 in which the carboxylic acid resin is a methacrylic acid polymer cross-linked with divinylbenzene.

9.   A composition as claimed in any one of claims 1 to 8 wherein the urease is bound to a portion of one of the zeolites present.

10.   A method for adsorption of uremic substances in an artificial kidney having semi-permeable membranes, said method comprising:

suspending with a suspending agent in water a mixture of components that includes a surface adsorptive agent capable of adsorption of uremic substances, urease, a cation exchanger and an aliphatic carboxylic acid resin in the $H^+$ form; and

contacting one side of said membranes with said suspension and the other side of said membranes with blood whereby uremic substances in said blood are dialyzed therefrom and then removed by said suspended mixture of components.

11.   A method of preparing a composition for use in dialysis comprising admixture of the components defined in claim 1.